# EUROPEAN PATENT SPECIFICATION

(11) **EP 0 311 260 B1**
(45) Date of publication and mention of the grant of the patent: **28.12.1994**
(21) Application number: 88308337.0
(22) Date of filing: 09.09.1988
(51) Int. Cl.: A61K 7/18, A61K 9/68

(54) **Oral compositions**
Orale Zusammensetzungen
Composition buccales

(30) Priority: 14.09.1987 US 97010
(43) Date of publication of application: 12.04.1989
(73) Proprietor: THE PROCTER & GAMBLE COMPANY, Cincinnati, Ohio 45202 (US)
(72) Inventor: Majeti, Satyanarayana, Cincinnati Ohio 45224 (US); Guay, Christopher Barry, Cincinnati Ohio 45240 (US); Crisanti, Mark Matthew, Cincinnati Ohio 45240 (US)
(74) Representative: Brooks, Maxim Courtney

(56) References cited:
- FR-A- 1 378 808
- FR-A- 2 406 437
- GB-A- 845 611
- US-A- 3 105 798
- US-A- 3 544 678
- US-A- 4 335 102
- Chemical Abstracts, vol. 82, no. 15, April 14, 1975, page 37, abstract 93144f, Colombus, Ohio, US, G.K. Stookey et al. "Cariostatic properties of tin (II) and oat hulls in the rat" & J. Dent. Res. 1974, 53(6), 1398-403

## Description

The present invention relates to oral compositions such as liquid dentifrices, toothpastes and mouthwashes, which provide antigingivitis benefits.

Plaque is recognized as a precursor of such oral diseases as caries and gingivitis. The gums of the mouth of humans and lower animals may be harmed by deposits of dental plaque, a combination of minerals and bacteria found in the mouth. The bacteria associated with plaque can secrete enzymes and endotoxins which can irritate the gums and cause an inflammatory gingivitis. As the gums become increasingly irritated by this process they have a tendency to bleed, lose their toughness and resiliency, and separate from the teeth, leaving periodontal pockets in which debris, secretions, more bacteria and toxins further accumulate. It is also possible for food to accumulate in these pockets, thereby providing nourishment for increased growth of bacteria and production of endotoxins and destructive enzymes. This can result in destruction of bone and gum tissue.

With such problems being possible from plaque/gingivitis it is not surprising that extensive efforts have been expended in trying to find effective treatment compositions. Many of these efforts have used quaternary ammonium compounds or bis-biquanides such as chlorhexidine which is used in Peridex® sold by The Procter & Gamble Company.

Another material which has been considered is stannous ion. Such a material is disclosed in Svatun B., "Plaque Inhibiting Effect of Dentifrices Containing Stannous Fluoride", Acta Odontol. Scand., 36, 205-210 (1978); and Bay I., and Rolla, G., "Plaque Inhibition and Improved Gingival Condition By Use of a Stannous Fluoride Toothpaste", Scand. J. Dent. Res., 88, 313-315 (1980).

US-A-3,105,798 discloses toothpaste compositions comprising stannous salts and gluconate salts, while GB-A-0,845,611 discloses toothpaste compositions comprising a source of fluoride and a stannous ion reservoir.

In spite of the many disclosures in the antiplaque/antigingivitis area, the need for improved products still exists. The present invention is directed to the recognition that stannous fluoride when used in combination with stannous gluconate provides improved efficacy. The gluconate salt provides an optimal stannous reservoir since stannous ions are lost during manufacture and storage.

It is an object of the present invention therefore to provide compositions which deliver an improved antigingivitis benefit.

It is a further object of the present invention to provide improved products utilizing stannous fluoride and stannous gluconate.

These and other objects will become clearer from the detailed description which follows.

All percentages and ratios used herein are by weight of the total composition unless otherwise specified. Additionally, all measurements are made at 25°C in the composition or in an aqueous solution/dispersion unless otherwise specified.

The present invention relates to an oral composition effective in treating gingivitis comprising:
(a) from 0.05% to 1.1% by weight of stannous fluoride;
(b) from 0.1% to 11% by weight of stannous gluconate; and
(c) a pharmaceutically acceptable carrier,
wherein the pH of said composition is from 3.0 to 5.0 and said composition is free of or comprises less than 2% by weight of a calcium ion source(s).

The compositions of the present invention comprise stannous fluoride and stannous gluconate and a pharmaceutically acceptable carrier.

By "oral composition" as used herein means a product which in the ordinary course of usage is not intentionally swallowed for purposes of systemic administration of particular therapeutic agents, but is rather retained in the oral cavity for a time sufficient to contact substantially all of the dental surfaces and/or oral tissues for purposes of oral activity.

By "safe and effective amount" as used herein means sufficient amount of material to provide the desired benefit while being safe to the hard and soft tissues of the oral cavity.

By the term "comprising", as used herein, is meant that various additional components can be conjointly employed in the compositions of this invention as long as the listed materials perform their intended functions.

By the term "carrier", as used herein, is meant a suitable vehicle which is pharmaceutically acceptable and can be used to apply the present compositions in the oral cavity.

### Stannous Fluoride

Stannous fluoride is the first essential component of the present compositions. This material is a staple item of commerce and is present in the present composition at a level of from 0.05% to 1.1%, preferably from 0.4% to 0.95%. It should be recognized that separate soluble stannous and fluoride salts may be used to form stannous fluoride in-situ as well as adding the salt directly. Suitable salts include stannous chloride and sodium fluoride among many others.

### Stannous Gluconate

Stannous gluconate is the second of the essential components of the present compositions. This material is a known stannous chelate and may be provided to the present compositions as the chelate or as separate soluble stannous and gluconate salts and the chelate formed in-situ such as with stannous fluoride. Such salts include stannous chloride and sodium gluconate. Stannous gluconate is present in the present compositions at a level of from 0.1% to 11%, preferably from 2% to 4%.

### Pharmaceutically Acceptable Carrier

The carrier for the stannous components can be any vehicle suitable for use in the oral cavity. Such carriers include the usual components of mouthwashes, toothpastes, tooth powders, prophylaxis pastes, gums and the like and are more fully described hereinafter. Dentifrices and mouthwashes are the preferred systems.

The abrasive polishing material contemplated for use in the present invention can be any material which does not excessively abrade dentin and do not provide calcium ions which may precipitate with, for example, the fluoride ions provided from stannous fluoride. These include, for example, silicas including gels and precipitates, insoluble sodium polymetaphosphate, β-phase calcium pyrophosphate, hydrated alumina, and resinous abrasive materials such as particulate condensation products of urea and formaldehyde, and others such as disclosed in US-A-3,070,510. Mixtures of abrasives may also be used. Abrasives such as calcium carbonate, calcium phosphate and regular calcium pyrophosphate are not used in the present compositions since they provide calcium ions which can complex F⁻.

Silica dental abrasives, of various types, can provide the unique benefits of exceptional dental cleaning and polishing performance without unduly abrading tooth enamel or dentin. Silica abrasive materials are also exceptionally compatible with sources of soluble fluoride. For these reasons they are preferred for use herein.

The silica abrasive polishing materials useful herein, as well as the other abrasives, generally have an average particle size ranging between about 0.1 and 30 µm, preferably 5 and 15 µm. The silica abrasive can be precipitated silica or silica gels such as the silica xerogels described in Pader et al. , US-A-3,538,230, issued March 2, 1970 and DiGiulio, US-A-3,862,307, June 21, 1975. Preferred are the silica xerogels marketed under the tradename "Syloid(RTM)" by the W. R. Grace & Company, Davison Chemical Division. Preferred precipitated silica materials include those marketed by the J. M. Huber Corporation under the tradename, "Zeodent (RTM)", particularly the silica carrying the designation "Zeodent (RTM) 119". These silica abrasives are described in US-A-4,340,583, July 29, 1982.

The abrasive in the compositions described herein is present at a level of from 6% to 70%, preferably from 15% to 25% when the dentifrice is a toothpaste. Higher levels, as high as 95%, may be used if the composition is a toothpowder.

Flavoring agents can also be added to dentifrice compositions. Suitable flavoring agents include oil of wintergreen, oil of peppermint, oil of spearmint, oil of sassafras, and oil of clove. Sweetening agents which can be used include aspartame, acesulfame, saccharin, dextrose, levulose and sodium cyclamate. Flavoring and sweetening agents are generally used in dentifrices at levels of from 0.005% to 2% by weight.

Dentifrice compositions can also contain emulsifying agents. Suitable emulsifying agents are those which are reasonably stable and foam throughout a wide pH range, including non-soap anionic, nonionic, cationic, zwitterionic and amphoteric organic synthetic detergents. Many of these suitable surfactants are disclosed by Gieske et al. in US-A-4,051,234 September 27, 1977,

It is common to have an additional water-soluble fluoride compound present in dentifrices and other oral compositions in an amount sufficient to give a fluoride ion concentration in the composition at 25°C and/or when it is used of from 0.0025% to 5.0% by weight, preferably from 0.005% to 2.0% by weight, to provide additional anticaries effectiveness. Preferred fluorides are sodium fluoride, indium fluoride, and sodium monofluorophosphate. Norris et al., US-A-2,946,735, issued July 26, 1960 and US-A-3,678,154, , issued July 18, 1972 disclose such salts as well as others.

Water is also present in the toothpastes of this invention. Water employed in the preparation of commercially suitable toothpastes should preferably be deionized and free of organic impurities. Water generally comprises from 10% to 50%, preferably from 20% to 40%, by weight of the toothpaste compositions herein. These amounts of water include the free water which is added plus that which is introduced with other materials such as with sorbitol.

In preparing toothpastes, it is necessary to add some thickening material to provide a desirable consistency. Preferred thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as gum karaya, gum arabic, and gum tragacanth can also be used. Colloidal magnesium aluminum silicate or finely divided silica can be used as part of the thickening agent to further improve texture. Thickening agents in an amount from 0.5% to 5.0% by weight of the total composition can be used.

It is also desirable to include some humectant material in a toothpaste to keep it from hardening. Suitable humectants include glycerin, sorbitol, and other edible polyhydric alcohols at a total level of from 15% to 70%.

Also desirable for inclusion in the toothpastes of the present invention are other stannous salts such as stannous pyrophosphate and antimicrobials such as quaternary ammonium salts, bis-biquanide salts, nonionic antimicrobial salts and flavor oils. Such agents are disclosed in US-A-2,946,735, July 26, 1960, to Norris et al. and US-A-4,051,234, September 27, 1977 to Gieske et al. These agents, if present, are included at levels of from 0.01% to 1.5%.

Another preferred embodiment of the present invention is a mouthwash composition. Conventional mouthwash composition components can comprise the carrier for the antimicrobial of the present invention. Mouthwashes generally comprise from 20:1 to 2:1 of a water/ethyl alcohol solution and preferably other ingredients such as flavor, sweeteners, humectants and sudsing agents such as those mentioned above for dentifrices. The humectants, such as glycerin and sorbitol give a moist feel to the mouth. Generally, on a weight basis the mouthwashes of the invention comprise 5% to 60% (preferably 18% to 25%) ethyl alcohol, 0% to 20% (preferably 5% to 20%) of a humectant, 0% to 2% (preferably 0.01% to 0.15%) emulsifying agent, 0% to 0.5% (preferably 0.005% to 0.06%) sweetening agent such as saccharin, 0% to 0.3% (preferably 0.03% to 0.3%) flavoring agent, and the balance water. The amount of additional antimicrobial agent in mouthwashes is typically from 0.01% to 1.5% by weight.

Suitable chewing gum components are disclosed in US-A-4,083,955, April 11, 1978 to Grabenstetter et al.

The pH of the present compositions is from 3.0 to 5.0, preferably from 4.0 to 5.0, most preferably about 4.5.

The carrier compositions of the present invention can be made using methods which are common in the oral products area. A specific method of manufacture is set forth in the Examples.

### EXAMPLES I - IV

The following dentifrice compositions are representative of the present invention.

| Component | Weight % | | | |
|---|---|---|---|---|
| | I | II | III | IV |
| Water | 12.500 | 12.500 | 12.500 | 12.500 |
| Sorbitol (70% Solution) | 47.891 | 45.727 | 43.437 | 41.328 |
| Glycerin | 10.198 | 10.198 | 10.198 | 10.000 |
| PEG(RTM)-12 | -- | -- | -- | -- |
| Titanium Dioxide | 0.525 | 0.525 | 0.525 | 0.525 |
| Silica | 20.000 | 20.000 | 20.000 | 20.000 |
| Na Carboxymethyl Cellulose | 1.050 | 1.050 | 1.050 | 1.000 |
| Na Carrageenan | -- | -- | -- | 0.350 |
| Magnesium Alumina Silicate | 0.408 | 0.408 | 0.408 | -- |
| Hydroxyethyl Cellulose | -- | -- | -- | -- |
| Na Alkyl Sulfate (27.9% Solution) | 4.000 | 4.000 | 4.000 | 4.000 |
| Na Gluconate | 0.632 | 2.395 | 4.790 | 5.514 |
| Stannous Fluoride | 0.454 | 0.454 | 0.454 | 0.454 |
| Stannous Chloride Dihydrate | -- | 1.141 | 1.141 | 2.198 |
| Stannous Pyrophosphate | 1.040 | -- | -- | -- |
| Na Saccharin | 0.200 | 0.200 | 0.200 | 0.230 |
| Flavor | 0.851 | 0.851 | 0.851 | 1.000 |
| FD&C Blue #1 (1% Solution) | 0.051 | 0.051 | 0.051 | 0.051 |
| Na Hydroxide (50% Solution) | 0.200 | 0.500 | 0.395 | 0.850 |
| pH | 4.5 | 4.5 | 4.5 | 4.5 |

### EXAMPLES V-VIII

The following dentifrice compositions are representative of the present invention.

| Component | Weight % | | | |
|---|---|---|---|---|
| | V | VI | VII | VIII |
| Water | 12.500 | 16.500 | 12.500 | 12.500 |
| Sorbitol (70% Solution) | 45.712 | 42.255 | 45.754 | 45.908 |
| Glycerin | 10.000 | 10.000 | 10.000 | 10.000 |
| PEG(RTM)-12 | -- | 3.000 | -- | -- |
| Titanium Dioxide | 0.525 | 0.525 | 0.525 | 0.525 |
| Silica | 20.000 | 20.000 | 20.000 | 20.000 |
| Na Carboxymethyl Cellulose | 1.000 | -- | 1.000 | 0.900 |
| Na Carrageenan | 0.350 | 0.450 | 0.350 | 0.350 |
| Magnesium Alumina Silicate | -- | -- | -- | -- |
| Hydroxyethyl Cellulose | -- | 0.400 | -- | -- |
| Na Alkyl Sulfate (27.9% Solution) | 4.000 | 4.000 | 4.000 | 4.000 |
| Na Gluconate | 2.082 | 2.395 | 2.395 | 2.082 |
| Stannous Fluoride | 0.454 | 0.454 | 0.454 | 0.908 |
| Stannous Chloride Dihydrate | 1.500 | 1.141 | 1.141 | 0.846 |
| Stannous Pyrophosphate | -- | -- | -- | -- |
| Na Saccharin | 0.230 | 0.230 | 0.230 | 0.230 |
| Flavor | 1.000 | 1.000 | 1.000 | 1.000 |
| FD&C Blue #1 (1% Solution) | 0.051 | 0.050 | 0.051 | 0.051 |
| Na Hydroxide (50% Solution) | 0.600 | 0.600 | 0.600 | 0.700 |
| pH | 4.5 | 4.5 | 4.5 | 4.5 |

### Procedure for Making Dentifrice

In preparing the dentifrice formulations, sorbitol and one half of the water are added to the mix tank and heating to 77°C initiated. Saccharin, titanium dioxide, and silica may be added to the mixture during this heating period. Sufficient agitation is maintained to prevent the settling of the insoluble components. The glycerin is added to a separate vessel and is also heated to 77°C. When both the solutions have attained the required temperature, the carboxymethyl cellulose (CMC) and carrageenan are blended together and slowly added to the glycerin under vigorous agitation. When the CMC and carrageenan are sufficiently dispersed in the glycerin, this mixture is added to the sorbitol/water mixture. The resulting mixture is then blended for a period of time sufficient to allow complete hydration of the binders (about 15 minutes). When the paste is of acceptable texture, the flavor, sodium alkyl sulfate, and color are added. One half of the remaining water is then added to a separate mix tank and allowed to heat to 77°C. After the water attains the necessary temperature, the sodium gluconate is added under medium agitation and allowed to dissolve completely. The stannous chloride dihydrate is then added to the gluconate solution and also allowed to dissolve. This mixture is added to the main mix. The stannous fluoride is added to the remaining water (also at 77°C) and the resulting solution is added to the main mix and allowed to blend thoroughly before final pH adjustment with sodium hydroxide. The completed paste is agitated for approximately 20 minutes before being milled and deaerated.

### EXAMPLES IX - XII

The following are mouthwash compositions representative of the present invention.

| Component | Weight % | | | |
|---|---|---|---|---|
| | IX | X | XI | XII |
| Stannous Fluoride | 0.100 | 0.100 | 0.100 | 0.100 |
| Stannous Chloride Dihydrate | 0.375 | 0.375 | 0.550 | 0.285 |
| Sodium Gluconate | 0.521 | 1.041 | 0.690 | 0.425 |
| Glycerin | 8.000 | 10.000 | -- | -- |
| Sorbitol (70% aqueous solution) | -- | -- | 10.000 | 12.000 |
| Ethanol | 10.000 | 10.000 | 10.000 | 10.000 |
| Polysorbate 80 | 0.300 | 0.300 | -- | -- |
| Sorbitan Diisostearate | -- | -- | 0.200 | 0.200 |
| Sodium Saccharin | 0.050 | 0.050 | 0.050 | 0.050 |
| Flavor | 0.150 | 0.150 | 0.150 | 0.150 |
| Sodium Hydroxide (50%) | 0.020 | 0.020 | 0.020 | 0.020 |
| Benzoic Acid | 0.050 | 0.050 | 0.050 | 0.050 |
| FD&C Blue #1 (1% solution) | 0.020 | 0.020 | 0.020 | 0.020 |
| Water | 80.414 | 77.894 | 78.170 | 76.700 |
| pH | 4.5 | 4.5 | 4.5 | 4.5 |

### EXAMPLE XIII

The following is a toothpowder representative of the present invention.

| Component | Weight % |
|---|---|
| Stannous Fluoride | 0.454 |
| Stannous Chloride Dihydrate | 1.500 |
| Sodium Gluconate | 2.082 |
| Silica | 74.964 |
| Sodium Sulfate | 20.000 |
| Sodium Lauryl Sulfate | 1.000 |
| Flavor | 1.000 |
| Sodium Saccharin | 0.200 |

### EXAMPLE XIV - XVI

The following are topical gels representative of the present invention.

| Component | XIV | XV | XVI |
|---|---|---|---|
| Stannous Fluoride | 0.454 | 0.454 | 0.454 |
| Stannous Chloride Dihydrate | 1.141 | 1.500 | 2.200 |
| Sodium Gluconate | 1.750 | 2.082 | 2.500 |
| Glycerin | 92.855 | 70.000 | 50.000 |
| Sorbitol (70% Solution) | -- | 21.964 | 42.146 |
| Sodium Carboxymethyl Cellulose | 0.600 | 0.800 | -- |
| Hydroxyethyl Cellulose | -- | -- | 0.500 |
| Flavor | 1.000 | 1.000 | 1.000 |
| Sodium Saccharin | 0.200 | 0.200 | 0.200 |
| Sodium Alkyl Sulfate (27.9%) | 2.000 | 2.000 | 1.000 |

### EXAMPLE XVII - XX

The following are mouthwash tablets representative of the present invention.

| Component | XVII | XVIII | XIX | XX |
|---|---|---|---|---|
| Stannous Fluoride | 0.100g | 0.100g | 0.100g | 0.100g |
| Stannous Chloride Dihydrate | 0.375g | 0.375g | 0.550g | 0.375g |
| Sodium Gluconate | 0.500g | 1.000g | 0.700g | 0.700g |
| Flavor | 0.150g | 0.150g | 0.150g | 0. 150g |
| Sodium Saccharin | 0.050g | 0.050g | 0.050g | 0.200g |
| Mannitol | 1.000g | -- | -- | -- |
| Sodium Carboxymethyl Cellulose | 0.050g | -- | -- | -- |
| Gum Arabic | -- | -- | 2.000g | -- |
| Corn Starch | -- | 2.000g | 0.500g | -- |
| Sodium Benzoate | 0.030g | 0.030g | 0.030g | 0.025g |
| Citric Acid | -- | -- | -- | 0.200g |
| Sodium Carbonate | -- | -- | -- | 0.100g |
| Sodium Bicarbonate | -- | -- | -- | 0.200g |
| Glycine | -- | -- | -- | 0.050g |

## Claims

1. An oral composition effective in treating gingivitis comprising:
(a) from 0.05% to 1.1% by weight of stannous fluoride;
(b) from 0.1 % to 11 % by weight of stannous gluconate; and
(c) a pharmaceutically acceptable carrier,
wherein the pH of said composition is from 3.0 to 5.0 and said composition is free of or comprises less than 2% by weight of a calcium ion source(s).

2. An oral composition according to Claim 1 wherein the pharmaceutically acceptable carrier is a toothpaste.

3. An oral composition according to Claim 2 which also contains a silica dental abrasive.

4. An oral composition according to Claim 3 which also contains another stannous salt.

5. An oral composition according to Claim 1 wherein the pharmaceutically acceptable carrier is a mouthwash.

6. An oral composition according to Claim 5 which also contains a material selected from the group consisting of a humectant, ethanol, a nonionic surfactant and mixtures thereof.

7. An oral composition according to Claim 5 wherein the pharmaceutically acceptable carrier is a mouthwash in tablet form.

8. An oral composition according to Claim 1 wherein the pharmaceutically acceptable carrier is a chewing gum.

9. Use of an oral composition for preparing a medicament for the treatment of gingivitis wherein the oral composition comprises:
a) from 0.05% to 1.1% by weight of stannous fluoride;
b) from 0.1 to 11% by weight of stannous gluconate; and
c) a pharmaceutically acceptable carrier,
and wherein the pH of said composition is from 3.0 to 5.0 and said composition is free of or comprises less than 2% by weight of a calcium ion.

## Patentansprüche

1. Bei der Behandlung von Gingivitis wirksame, orale Zusammensetzung, umfassend:
(a) 0.05 bis 1,1 Gew.-% Zinn(II)-fluorid;
(b) 0.1 bis 11 Gew.-% Zinn(II)-gluconat: und
(c) einen pharmazeutisch annehmbaren Träger,
wobei der pH der Zusammensetzung 3.0 bis 5,0 beträgt und die Zusammensetzung frei ist an oder weniger als 2 Gew. -% an Calciumionenquelle(n) umfaßt.

2. Orale Zusammensetzung nach Anspruch 1, wobei der pharmazeutisch annehmbare Träger eine Zahnpaste ist.

3. Orale Zusammensetzung nach Anspruch 2, welche ebenso ein Dental-Siliciumdioxidscheuermittel enthält.

4. Orale Zusammensetzung nach Anspruch 3, welche ebenso ein weiteres Zinn(II)-salz enthält.

5. Orale Zusammensetzung nach Anspruch 1, wobei der pharmazeutisch annehmbare Träger ein Mundspülmittel ist.

6. Orale Zusammensetzung nach Anspruch 5, welche ebenso ein Material enthält, gewählt aus der ein Feuchthaltemittel, Ethanol, ein nichtionisches oberflächenaktives Mittel und Mischungen davon umfassenden Gruppe.

7. Orale Zusammensetzung nach Anspruch 5, wobei der pharmazeutisch annehmbare Träger ein Mundspülmittel in Tablettenform ist.

8. Orale Zusammensetzung nach Anspruch 1, wobei der pharmazeutisch annehmbare Träger ein Kaugummi ist.

9. Verwendung einer oralen Zusammensetzung zur Herstellung eines Arzneimittels zur Behandlung von Gingivitis, wobei die orale Zusammensetzung
(a) 0,05 bis 1.1 Gew.-% Zinn(II)-fluorid;
(b) 0,1 bis 11 Gew.-% Zinn(II)-gluconat; und
(c) einen pharmazeutisch annehmbaren Träger, umfaßt
und wobei der pH der Zusammensetzung 3,0 bis 5,0 beträgt und die Zusammensetzung frei ist oder weniger als 2 Gew.-% an Calciumionenquelle(n) umfaßt.

## Revendications

1. Composition buccale efficace dans le traitement de la gingivite comprenant:
(a) de 0_{,}05% à 1_{,}1% en poids de fluorure stanneux;
(b) de 0,1% à 11% en poids de gluconate stanneux; et
(c) un support pharmaceutiquement acceptable,
dans laquelle le pH de ladite composition est de 3,0 à 5,0 et ladite composition est exempte de ou comprend moins de 2% en poids d'une source (ou de sources) d'un ion calcium.

2. Composition buccale selon la revendication 1 dans laquelle le support pharmaceutiquement acceptable est une pâte dentifrice.

3. Composition buccale selon la revendication 2 contenant également un abrasif dentaire siliceux.

4. Composition buccale selon la revendication 3 contenant également un autre sel stanneux.

5. Composition buccale selon la revendication 1 dans laquelle le support pharmaceutiquement acceptable est un bain de bouche.

6. Composition buccale selon la revendication 5 contenant également une substance choisie dans le groupe constitué par un humectant, l'éthanol, un agent tensioactif non ionique et leurs mélanges.

7. Composition buccale selon la revendication 5 dans laquelle le support pharmaceutiquement acceptable est un bain de bouche sous forme de comprimé.

8. Composition buccale selon la revendication 1 dans laquelle le support pharmaceutiquement acceptable est un chewing gum.

9. Utilisation d'une composition buccale pour préparer un médicament pour le traitement de la gingivite dans lequel la composition buccale comprend:
a) de 0,05% à 1,1% en poids de fluorure stanneux;
b) de 0,1% à 11% en poids de gluconate stanneux; et
c) un support pharmaceutiquement acceptable,
et dans lequel le pH de ladite composition est de 3,0 à 5,0 et ladite composition est exempte de ou comprend moins de 2% en poids d'un ion calcium.
